# EUROPEAN PATENT SPECIFICATION

(11) **EP 2 869 810 B1**
(45) Date of publication and mention of the grant of the patent: **07.03.2018**
(21) Application number: 12756827.7
(22) Date of filing: 05.07.2012
(51) Int. Cl.: A61K 8/66, A61K 38/43, A61Q 17/04, A61P 17/18

(54) **COMPOSITIONS FOR PREVENTING AND REPAIRING SKIN AND OCULAR DAMAGES CAUSED BY UV RADIATIONS**
ZUSAMMENSETZUNGEN ZUR VORBEUGUNG UND REPARATUR VON HAUT- UND AUGENSCHÄDEN DURCH UV-STRAHLUNG
COMPOSITIONS POUR PRÉVENIR ET RÉPARER DES LÉSIONS CUTANÉES ET OCULAIRES CAUSÉES PAR DES RAYONNEMENTS ULTRAVIOLETS (UV)

(43) Date of publication of application: 13.05.2015
(73) Proprietor: Biodue S.p.A., 50028 Tavarnelle Val di Pesa (FI) (IT)
(72) Inventor: BENEDETTI, Ruffo, I-50028 Tavarnelle Val di Pesa (FI) (IT)
(74) Representative: Perani & Partners S.p.A.
(86) International application number: PCT/IT2012/000206
(87) International publication number: WO 2014/006645

(56) References cited:
- WO-A1-2004/004673
- WO-A1-2006/041526
- WO-A1-2012/149110
- WO-A2-03/039478
- US-A1- 2004 057 917
- US-B2- 7 892 523
- OHRESSER S ET AL: "VALIDATION OF MICROBIAL RECOVERY FROM HYDROGEN PEROXIDE-STERILIZED AIR", PDA JOURNAL OF PHARMACEUTICAL SCIENCE AND TECHNOLOGY, BETHESDA, MD, US, vol. 58, no. 2, 1 March 2004 (2004-03-01), pages 75-80, XP008044984, ISSN: 1079-7440

## Description

### FIELD OF THE INVENTION

The present invention relates to enzyme-based compositions and to their uses for preventing and/or repairing skin damages caused by UV radiations.

### BACKGROUND OF THE INVENTION

Exposure of skin to ultraviolet (UV) radiations may cause photodamage of DNA, in particular UVB rays (290-320 nm) have a direct mutagenic action on DNA and UVB (320-400 nm) have indirect mutagenic action through formation of free radicals. The outcomes of these damages range from photo-aging to mutation, apoptosis and carcinogenesis (Marrot, L. and Meunier, J.R. "Skin DNA photodamage and its biological consequences", J. Am. Acad. Dermatol. 2008, 58 (Suppl 2): 139-148).

In particular, prolonged exposure of a field of tissue to UV radiations may trigger a constellation of locoregional changes, known as field carcinogenesis, which may lead to various types of skin cancer including melanoma. This is particularly relevant for individual which are frequently exposed to sunlight without adequate protection due to professional reasons (outdoor workers) or to psychological factors (e.g. tanning addiction or "tanorexia", a syndrome where an individual appears to have a physical or psychological addiction to sunbathing or to the use of tanning beds).

Photoprotection aims to preventing photodamage of DNA due to esposure to UV radiations, in particular to direct and prolonged exposure to sunlight. Treatment of damages caused by UV radiations may be effected by repairing DNA alterations in skin tissues at molecular level, for example via topical application of xenogenic DNA-repairing enzymes. Photolyases (EC 4.1.99.3) are monomeric flavoenzymes (MW 50-60kDA) involved in the mechanism of DNA repair against the damages caused by UV radiations. Photolyase is a phylogenetically ancient enzyme which is present and functional in several species ranging from the bacteria to the fungi to some animals but is not functional in placental mammals, including humans.

Photolyases bind complementary DNA strands and break certain types of pyrimidine dimers that arise when a pair of thymine or cytosine bases on the same strand of DNA become covalently linked. These dimers result in a 'bulge' of the DNA structure, referred to as a lesion. The most common covalent linkage involves the formation of a cyclobutane bridge. Photolyases have a high affinity for these lesions, which they reversibly bind and convert back to the original bases.

However, photolyases themselves require photoactivation, i.e. the presence of visible or near-UV light, to exert their DNA repairing action (Carell, T, et al. "The mechanism of action of DNA photolyases" Curr Opin Chem Biol. 2001; 5(5), 491-8; Essen, L. O.; Klar, T. Cell. Mol. Life Sci. 2006, 63, 1266) .

For this reason, although some photolyases have been used as a component of sunscreen formulations, in the prior art the DNA repair mechanism of photolyases is deemed not be active in the presence of a complete barrier to sunrays.

Hofer, A. et al. (Photochem.Photobiol. Sci. 2011, 10, 1118) describe studies for the use of DNA-repair enzymes in the treatment of polymorphic light eruption (PLE, a type of photodermatosis) comprising exposure to UV radiations, application of photolyases and subsequent exposure to visible blue light to activate the DNA repair mechanism of photolyase and visual evaluation of the PLE lesions morphology. Thus, further exposure to light, albeit potentially aggravating the damages of radiation exposure, appears necessary to benefit from the photolyase activity.

Endonucleases (EC 3.1.31.1) are another class of DNA-repairing enzymes that are commonly found in prokariotes (bacteria and archaea), and in some rare cases are produced by some eukaryotic organisms. To the contrary of photolyases, the DNA repair mechanism of endonucleases does not require the presence of light, although endonucleases are reportedly less effective than photolyases in damage repair (Stege, H. J. Photochem. Photobiol. B. 2001, 65, 105).

Hofer et al (Photochem.Photobiol. Sci. 2011, 10, 1118) also report that the compositions comprising liposomal DNA-repair enzymes do not have sunburn protection as their SPF (solar protection factor) is of only 1.

An object of the present invention is to provide an efficient method of conjugating photoprotection and DNA repair in a subject in need thereof.

Another aim of the present invention is to provide a combination of compositions to reverse the degeneration of UV-caused field carcinogenesis into cancers.

Another aim of the present invention is to provide a composition to prevent photo-induced skin damages also by application on a subject after exposure to UV radiations without adequate protection.

### BRIEF DESCRIPTION OF THE FIGURES

Figure 1 relates to the reduction of UV-induced formation of thymine dimers (CPDs) following application of the kit of the invention.
Figure 2 relates to the reduction in degree of UV-induced apoptosis following application of the kit of the invention.
Figure 3 relates to protection against UV-induced reduction of telomeres length following application of the kit of the invention.
Figure 4 relates to the reduction of the FOS gene expression following application of the kit of the invention.

### SUMMARY AND DESCRIPTION OF THE INVENTION

The present invention fulfils the above aims by the following.

The present invention provides for a kit comprising:
i. a sunscreen composition in form of a cream or of an oil/water emulsion with sun protection factor at least 50 which comprises at least one UV-screening compound and liposomes including photolyase from *Anacystis nidulans;*
ii. a composition in form of a cream or of an emulsion comprising liposomes including endonuclease from *Micrococcus luteus.*

In one aspect, the present invention relates to said kit for use in the treatment and/or prevention of damages induced by exposure to UV radiations, wherein the treatment and/or prevention comprises the topical application, on the body areas to be exposed to the UV radiation, of composition i. prior to exposure to the radiations and the topical application of composition ii. on the same parts after exposure to UV radiation.

Furthermore, it is herewith described a liquid composition for topical application, which is not part of the present invention, comprising liposomes including photolyase from *Anacystis nidulans* and endonuclease from *Micrococcus luteus* and sodium piruvate and to said sprayable composition for use in prevention and treatment of UV-induced photodamage and inflammation to the eye and of the ocular area. Furthermore is herewith disclosed a composition in the form of a cream or of an emulsion, which is not part of the present invention, comprising endonuclease from *Micrococcus luteus* for use in the prevention of damages caused by exposure to sunlight or UV radiations without adequate protection (tanning addiction) wherein the prevention comprises topical application of the composition to the body areas that have been exposed to the sunlight/radiations.

As used herein, the term "emulsion" refers to all types of emulsions of tow or more immiscible liquids, for example comprising oil in water, water in oil, oil/water/oil etc., which are suitable for topical application on skin.

As used herein, "photolyase from *Anacystis nidulans"* and "endonuclease from *Micrococcus luteus"* may refer to the single enzyme as well as to an extract from the respective microorganism which comprises the DNA repair enzyme.

As used herein, "liposomes" may refer to any type of artificially-prepared vesicle composed of a lipid bilayer suitable for pharmaceutical preparations as known to the person skilled in the art.

In an aspect, the present invention relates to a kit comprising:
i. a sunscreen composition in form of a cream or of an emulsion with sun protection factor at least 50 which comprises at least one UV-screening compound and liposomes including photolyase from *Anacystis nidulans;*
ii. a composition in form of a cream or of an oil/water emulsion comprising liposomes including endonuclease from *Micrococcus luteus.*

It was surprisingly found that topical application of the compositions in the kit of the invention reduces damages to the skin which are caused by exposure to sunlight or to other sources of UV radiations. In fact, it was found that the DNA alterations caused by UV radiations are greatly reduced after topical application of the compositions in the kit of the invention.

UVB radiations induce the formation of two main photoproducts: cis-syn cyclobutane pyrimidine dimers (thymine dimer, hereafter CPDs) and pyrimidine-pyrimidone (6-4) photoproducts (6-4 PPs). These lesions block DNA replication and transcription and are reportedly one of the major factors in the formation of skin cancers (Lima-Bessa, K. M.; Menck, C.F.M. Curr. Biol., 2005, 15, R58-R61).

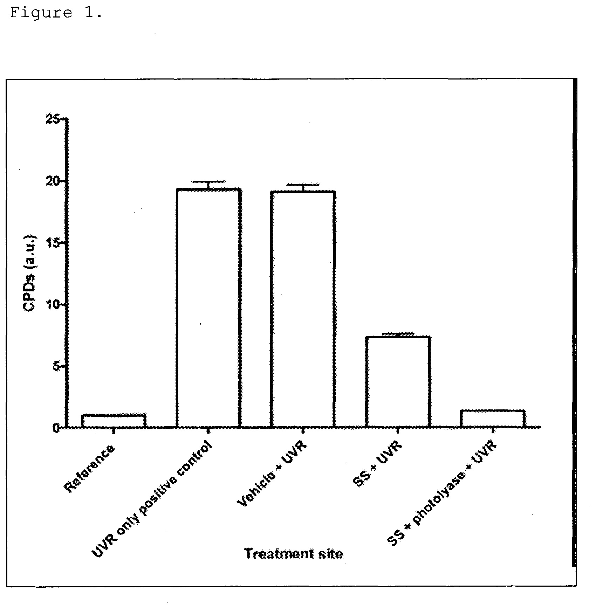

After irradiation for 4 consecutive days, it was found (Figure 1) that application of the composition i. of the invention, comprising photolyases and a sunscreen with sun protection factor (SPC) 50, reduces the number of CPDs by 93%, whereas a sunscreen with SPC 50 alone only reduces CPDs formation by 62%.

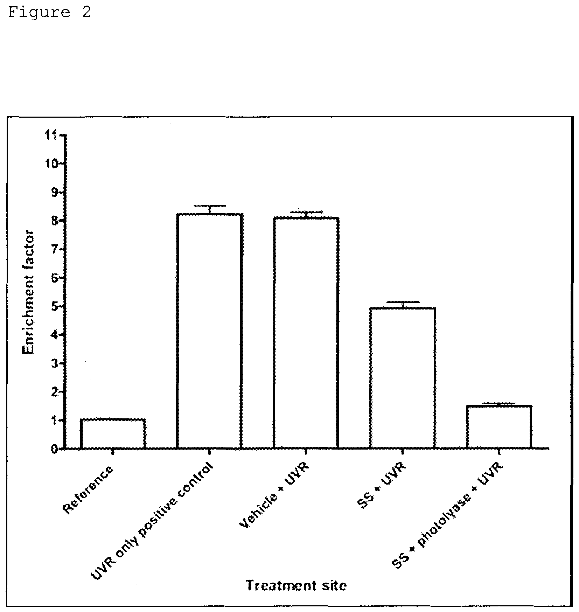

Under the same experimental conditions (Figure 2), it was found that the application of the composition i. of the invention greatly reduces UVB-mediated apoptosis of skin cells (a indicator of intervened cellular photodamage) also with respect to the application of a SPF 50 sunscreen alone.

These results are particularly unexpected considering that the prior art teaches that photolyases necessarily require the presence of visible light to exert their DNA-repairing activity. Because SPF 50 (or higher) sunscreens are regarded as total blockers of UV radiation and as strong blockers of visible light, it is surprising that the photolyases are found active in DNA repairing also in the presence of said filters.

A remarkable advantage of these findings is that it is possible to combine in a single product a sunscreen and the photolyase, thus providing strong sun protection together with simultaneous DNA repair action. Said product may be applied also prior or during sun exposure, to obtain maximum protection against photodamage via DNA-repair and prevention of sunburns, together with ease of application.

This is in evident contrast with the teachings of the prior art, wherein the use of photolyases is considered as compatible with the simultaneous application of moderate to mild sunscreens only. Moreover, it was surprisingly found that the application of the kit of the invention prevents reduction of the telomeres length in UV-exposed skin. Telomeres form the terminal region of the chromosomes and consist of highly repeated DNA sequences. Telomeres are of paramount importance in preserving the integrity of genetic code as DNA polymerase cannot continue their duplication fully to the end of chromosome. The telomeres, which may be considered as disposable buffers blocking the ends of the chromosomes, are consumed during cell division and are replenished by a different enzyme, namely telomerase reverse transcriptase.

The progressive reduction of telomeres size is linked to cellular aging and to the risk of developing neoplasia. In fact, telomeres of reduced size are a known biomarker of impaired genomic integrity.

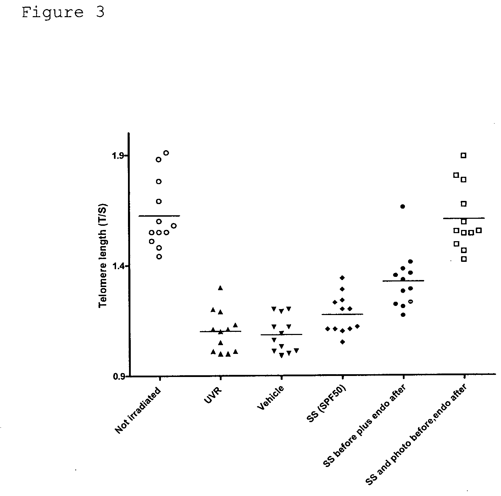

It was determined that repeated exposure to UV light reduces the length of telomeres in exposed skin tissues also in the presence of a SPF 50 (or higher) sunscreen filter. However, application of the composition ii. of the kit of the invention (comprising endonuclease of *Micrococcus luteus*) improves the results obtained in the presence of a SPF50 sunscreen (Figure 3).

Even more remarkable are the results obtained applying composition i. (SPF at least 50 sunscreen + photolyase) before exposure, followed by the application of composition ii. (endonuclease) after exposure (data on the farther right of Figure 3). In this case, the length of telomers is practically unaltered with respect to a control area unexposed to the UV radiation.

Thus, application of the kit of the present invention actually protects the skin tissue from photodamage to the telomeres and reduces the related risk of neoplasm formation.

In another experiment , the effect of the kit of the invention on the expression of FOS gene is investigated.

The FOS (Finkel-Biskis-Jinkins Osteosarcoma) gene is an oxidative stress marker which is an indicator of potential carcinogenesis.

It was reported (Saez, E. et al. Cell, 1995, 82, 721)that FOS is required for malignant progression of skin tumors, as FOS-deficient mice fail to undergo malignant progression of skin tumors in a transgenic model of oncogenic Ras.

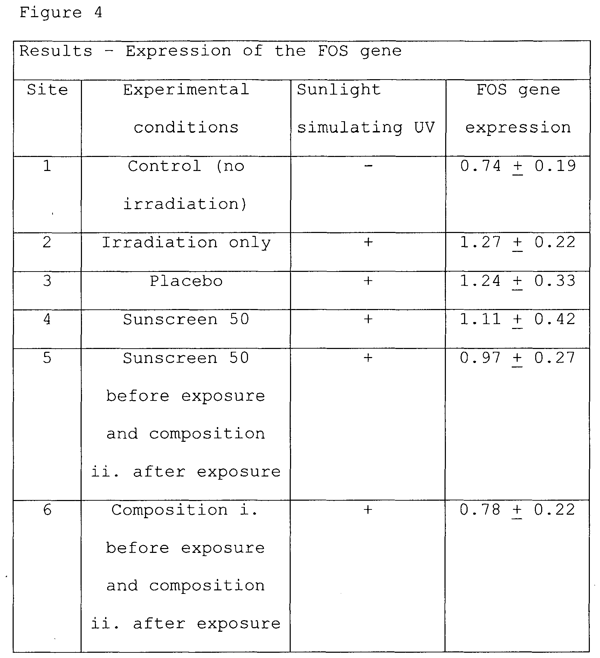

Remarkably, after application of the composition ii. of the kit of the invention (comprising endonuclease of *Micrococcus luteus*) following exposure in the presence of a SPF50 sunscreen, the expression of the FOS gene is lower than in the presence of a SPF50 sunscreen alone (Figure 4).

Application of the composition i. of the invention (SPF at least 50 sunscreen and photolyase) prior to exposure to radiations followed by application of the composition ii. (endnuclease) after exposure completely inhibits extra production of the FOS gene, in that the FOS level after exposure are substantially identical to that in the unexposed skin tissue.

Thus, topical application of the kit according to the present invention substantially repairs and prevents the adverse effects of UV radiations on telomeres and on the expression of the FOS gene.

Suitable UV-screening compounds for the composition of the invention comprise 2-cyano-3,3-diphenylacrylic acid, 2-ethylhexylic ester (octocrilene), N-{(2 e 4)-[2-oxo-born-3-ylidenmethyl] benzyl} acrilamide polymer, 2-ethylhexyl 4-ethoxycynnamate, ethoxylates 4-aminobenzoic acid, amyl 4-methoxycinnamate (isomeric mixture), 2-phenylbenzimidazol-5-sulphonic acid, 3,3'-(1,4-phenylenedimethylene) bis [7,7-dimethyl-2-oxobicyclo[2.2.1)hepta-1-yl) methanesulfonic acid and its salts, 1-(4-tert-butylphenyl)-3-(4-methoxiphenyl)-1 ,3-propandione, alpha-(2-oxoborn-3-yliden)toluen-4-sulfonic acid and its salts; 2,4,6-trianilino-(p-carbo-2'-ethylhexyl-1'-oxy)-1,3,5-triazine, benzoic acid 2-(2H-benzotriazol-2-yl)-4-methyl-6-[2-methyl-3-[1,3,3,3-tetramethyl-1-[(trimethylsilyl)oxi]disiloxanyl] propyl]phenol-4,4-[[6-[[4-[[(1,1-dimethylethyl)amino] carbonyl]phenyl]amino]-1,3,5-triazin-2,4-diyl]diimino] bis,bis(2-ethylhexyl) ester, 3-(4-methylbenzyliden)bornanone; 3-benzylidenbornanone; 2-ethylhexyl salicilate; 2-ethylhexyl-4-dimethylaminobenzoato, Sulisobenzone (DCI) and its sodium salt; 2,2' -methylene-bis-[6-(2H-benzotriazol-2-il)-4-(1,1,3,3-tetramethylbutil)]phenol, 2,2'-(1,4-phenylen)bis-1H-benzimidazol-4,6-disulfonic acid sodium salt; 2,2'-[6-(4-methoxyphenyl)-1,3,5-triazine-2,4-diyl]bis[5-(2-ethylhexyl)oxi)]phenol, dimethicodiethylbenzalmalonate, Titanium dioxide, hexyl 2-[4-(diethylamino)-2-hydroxibenzoyl]-benzoate (INCI name: Diethylamino Hydroxybenzoyl Hexyl Benzoate), N,N,N-trimethyl-4-[(2-keto-3-bornilidene)methyl] aniline metylsulfate; Homosalate (DCI), Oxybenzone (DCI) and mixtures thereof. Preferably, in the kit according to the present invention the at least one UV-screening compound of composition i. is selected from the group consisting of methylene bis-benzotriazolyl tetramethylbuthylphenol, ethylhexyl methoxycinnamate, bis-ethylhexyloxyphenol methoxy phenyltriazine, butyl methoxydibenzoylmethane, 2-ethylhexyl-2-cyano-3,3-diphenylacrylate and mixtures thereof.

Preferably, in the kit according to the invention the composition i. comprises liposomes-included photolyase from *Anacystis nidulans* in a concentration between 0.1% and 5% w/w and the composition ii. comprises liposomes-included endonuclease from *Micrococcus luteus* in a concentration between 0.1% and 5% w/w.

The compositions of the present invention may comprise additional components typically used in composition for topical administration as known to the person skilled in the art such as vitamins, peptides, film-forming agents, emulsifiers, viscosity modifiers, thickeners, dispersant, pH-modifiers, perfumes, preservatives, chelating agents and their mixtures.

In another aspect, the present invention relates to the kit comprising the compositions i. and ii. as defined above for use in the treatment and/or prevention of damages induced by exposure to UV radiations, wherein the treatment and/or prevention comprises the topical application, on the body areas to be exposed to the UV radiation, of composition i. prior to exposure to the radiations and the topical application of composition ii. on the same parts after exposure to UV radiation.

Preferably, said kit is for use in treatment and/or prevention of field cancerization induced by exposure to sunlight or to other sources of UV radiation. Field cancerization may be in the form of actinic keratosis and squamous cell carcinoma in subjects with chronic (frequent and/or prolonged) exposure to sunrays and/or UV radiations.

Application of the kit of the invention on areas affected by actinic keratosis and squamous cell carcinoma may result in partial to total recovery from the lesions with up to full tissue repair. For example, partial recovery has been observed in the case of squamous cell carcinoma.

The kit of the invention is also useful for secondary prevention of cancer recurrence on field cancerization in subjects with previous basal-cell carcinoma.

Preferably, the kit according to the present invention is for use in the prevention of damages caused by prolonged exposure to sunlight or to UV radiations without adequate protection (e.g. in tanning addiction or "tanorexia"), by prolonged exposure to sunlight in outdoor workers, by photodynamic treatment (PDT), by exposure to sunlight of subjects with Fitzpatrick phototype II, suffering from immunodeficiency or photoallergy (photodermatitis), under prolonged cancer or steroidal therapy, or with tendency to multiple or recurrent basal-cell carcinoma.

In another aspect, is herewith disclosed a liquid composition suitable for topical application, not belonging to the present invention, comprising photolyase from *Anacystis nidulans* and endonuclease from *Micrococcus luteus* and sodium piruvate.

It was found that topical application of the association of photolyase and endonuclease with sodium pyruvate on the ocular area actively provides an advanced molecular protection of the eye, in particular of cornea, of crystalline lens and of retina against UV- and sunlight-caused photodamage and prevents/treats inflammation in the anterior chamber.

The liquid composition, which is not object of the present invention, may be applied in spray form or in drop form, for example in eyedrop form.

Examples of diseases/condition which may be prevented or treated with the liquid composition not belonging to the present invention are AMD (age-related macular degeneration), cataract, pterygium, photokeratitis ("welder's flash"), computer eye strain and actinic congiuntivitis.

Preferably, in said liquid composition, not belonging to the invention, the concentration of photolyase is 0.5% w/w and the concentration of endonuclease is 0.5% w/w.

Furthermore, it is disclosed the use of said liquid composition in prevention and treatment of UV-induced photodamage and inflammation to the eye and of the ocular area.

In another aspect, it is herewith described a composition comprising endonuclease from *Micrococcus luteus,* not belonging to the present invention, for use in the prevention of damages caused by exposure to sunlight or UV radiations without adequate protection (tanning addiction) wherein the prevention comprises topical application of the composition to the body areas that have been exposed to the sunlight/radiations.

It was surprisingly found that the application of the composition comprising endonuclease from *Micrococcus luteus* of the present invention before and/or after exposure is particularly efficient in preventing the damages caused by excessive exposure to sunlight, and to UV radiations in general, without proper protection, for example by means of a sunscreen.

This feature may be of interest for subject, such as the so called "tanning addicts" or "tanorexics", which deliberately avoid any form of protection during exposure to direct sunlight or to tanning radiations, but may comply with a schedule of prevention comprising topical application of a repair product following exposure.

Preferably, the composition comprising endonuclease from *Micrococcus luteus,* not belonging to the present invention, is applied before and after exposure to sunlight and to Uv radiations in general.

The following examples are intended to illustrate specific embodiments of the invention, without limiting its scope.

### Examples

### Example 1

The following study aims at evaluating the molecular outcomes of topical application of a SPF AT LEAST 50 sunscreen and of a composition (composition i.) comprising a SPF AT LEAST 50 sunscreen and photolyase from the cyanobacter *Anacystis nidulans* in liposomal form.

The effects are measured in terms of formation of CPDs and of the degree of UV radiation-induced apoptosis.

### Subjects

Ten healthy Caucasian volunteers (5 males, 5 females, age range 25-36) with Fitzpatrick skin II are recruited excluding subjects with a history of photodermatosis and skin cancer.

No subject taking any photosensitizing or antiinflammatory medications is admitted to the tests.

### Materials

Ten healthy Caucasian volunteers (5 males, 5 females, age range 25-36) with Fitzpatrick skin II are recruited excluding subjects with a history of photodermatosis and skin cancer.

No subject taking any photosensitizing or antiinflammatory medications is admitted to the tests.

### Materials

The sunscreen (sun protection factor at least 50) contains methylene bis-benzotriazolyl tetramethylbuthylphenol (Tinosorb M™), 50% solution (4%), ethylhexyl methoxycinnamate (Parsol MCX™) (8%), bis-ethylhexyloxyphenol methoxy phenyltriazine (Tinosorb S™) (5%), butyl methoxydibenzoylmethane (Eusolex 9020™) (2%) and 2-ethylhexyl-2-cyano-3,3-diphenylacrylate (Eusolex OCR™) (1%).

The photolyase preparation contains the same filters plus photolyase derived from the cyanobacterium *Anacystis nidulans* in a liposomal preparation (1%). The vehicle is a commercially available moisturizer base.

### Solar simulator

The solar-simulated radiation is produced by an Oriel solar simulator (Model 81292; L.O.T. Oriel, Leatherhead, UK) containing a 1 kW xenon arc lamp with two dichroic mirrors, a collimator and a 1-mm WG320 filter. The optical design of this particular solar simulator gives a field of even irradiance (290-400 nm) to the skin surface when positioned 11 cm from the source, of which ∼10% is UVB (280-320 nm) and the remainder UVA. The spectral irradiance is measured with an OL754 spectroradiometer (Optronics, Orlando, FL, USA), calibrated for wavelength and intensity against standard lamps. The spectroradiometer is used to calibrate a handheld IL700 radiometer (International Light, Newburyport, MA, USA), which is then used to monitor rapidly lamp output on a daily basis.

### Irradiation and treatment protocol.

Two weeks prior to the test irradiations, the minimal erythema dose (MED) is determined for each individual for solar-simulated UVR (290-400 nm) and expressed in mJ/cm² using a light-proof adhesive-backed foil template that is sequentially uncovered to deliver quantities of UV above and below the expected MED of skin phototype II individuals for solar-simulating UVR. The sites are examined 24 h after irradiation and the MED is determined as the site that showed minimal, uniform perceptible erythema.

Before irradiation, five circular areas (10 mm diameter) were marked out on the non-exposed lower back of each participant.

On four consecutive days, four sites (designated sites 2-5) were exposed to solar-simulated (ss) UVR at three times MED.

### Site 1 received no ssUVR (reference).

Thirty minutes prior to each irradiation, the following products were applied to sites 3-5, respectively:
- vehicle (moisturizer base cream),
- SS (sunscreen) alone and
- SS plus photolyase.

No product is applied to site 2 (UVR only).

Subjects reported to the study center for all irradiations, and all test product applications are performed by the investigators. Seventy-two hours following the last exposure to ssUVR, skin specimens are obtained through a 4-mm punch biopsy from all sites for molecular analyses.

### DNA extraction.

The skin biopsy specimens are cleaved in half, and one piece is thawed at room temperature, minced and lysed by 3 cycles of freezing (in an ethanol-dry-ice bath) and thawing (at 95°C).

Samples are digested for 12 h at 60°C with proteinase K in 100 mmol/l Tris-HCl (pH 7.4), 150 mmol/l NaCl and 10 mmol/l EDTA (pH 8.0). Proteinase K is heat-inactivated at 95°C for 10 min, and homogenates are extracted using the Puregene DNA Isolation kit (Gentra Systems, Minneapolis, MN, USA).

The kit contains two main reagents: cell lysis and protein precipitation solutions.

In brief, DNA is extracted from homogenates using a lysis buffer solution and then treated with RNase A. The kit removes proteins using a precipitation solution, followed by 2-propanol to pellet the DNA.

### CPDs.

Determination of CPDs in DNA extracted from homogenates is performed by ELISA, as described in Sancar, A. J. Biol. Chem. 2008, 283, 32153.

Briefly, purified DNA is diluted to 0.6 µg/ml in 2X saline-sodium citrate (SSC) buffer, and then denatured by boiling for 5 min. Samples are placed on ice, and 100 µl of each is added to 1% protamine sulfate-treated wells in 96-well ELISA plates.

The primary antibody (catalog no. MC-062; Kamiya Biomedical Company, Seattle, WA, USA) is diluted 1:2,000 to 0.25 µg/ml in 0.05% Tween-20/0.25% bovine serum albumin (BSA)/1X phosphate-buffered saline (PBS) blocking solution. An alkaline phosphatase conjugate (catalog no. AP130A; Chemicon, Temecula, CA, USA) diluted 1:10,000 in blocking solution is used as secondary antibody. Following incubation with the secondary antibody, wells are washed with 1X PBS, and CPDs are quantified with a nitrophenyl phosphate disodium-substrate assay.

Absorbance is read at 405 nm.

Each experiment is performed in duplicate, and the results are averaged. Higher absorbance is associated with higher CPDs. The results are plotted in arbitrary units relative to the values of the baseline control site.

The effect of a sunscreen with or without photolyase on CPD formation after repetitive UVR exposure on human skin *in vivo* is depicted in Figure 1. Repetitive irradiation significantly increased the formation of CPDs in both the UVR only positive control (site 2) and vehicle + UVR, site 3 (19-fold higher at both sites compared to baseline site 1, P<0.001). The application of SPF AT LEAST 50 sunscreen alone (site 4) significantly, but not completely, prevented CPD formation, reducing it by 62% (P<0.001 vs. UVR only positive control and vehicle + UVR sites). However, application of composition i. (topical SS + photolyase, site 5) prevents CPD formation by ∼93% and has a significantly superior effect with respect to SS alone (site 4, P<0.001).

### Apoptosis

Figure 2 shows the effect of a sunscreen (SS) with or without photolyase on apoptosis in skin biopsies after repetitive ultraviolet radiation (UVR) exposure obtained as described above.

ANOVA followed by Newman-Keuls tests is used to analyze apoptosis. Repetitive irradiation significantly increases apoptosis in both the UVR only positive control (site 2) and vehicle + UVR sites (site 3, P<0.001 vs. baseline site 1). Sunscreen alone (site 4) significantly, but not completely, prevents apoptosis (P<0.001 vs. UVR only positive control, site 2, and vehicle + UVR, site 3). However, topical application of SS + photolyase (site 5) has a significantly superior effect with respect to SS alone (site 4, P<0.001).

### Example 2

The following study aims at evaluating the molecular outcomes of topical application of a SPF AT LEAST 50 sunscreen and of a composition comprising liposomes including endonuclease from *Micrococcus luteus* (composition ii.) alone or following application of a composition comprising a SPF AT LEAST 50 sunscreen and photolyase from the cyanobacter *Anacystis nidulans* in liposomal form (composition i.).

### Subjects

Twelve healthy Caucasian volunteers (6 males and 6 females, age range: 26 to 31 years) with Fitzpatrick skin type I-II were recruited for the study. None of the subjects used any medication or had a history of photodermatosis and skin cancer.

The study was conducted in winter to minimize the effect of ambient sun exposure. The study protocol was approved by the local Medical Research Ethics Committee and written informed consent was obtained from each subject before participation in the study.

### Test materials

The SS (sun protection factor [SPF] 50) contained Tinosorb M, 50% solution (4%), Parsol MCX (8%), Tinosorb S (5%), Eusolex 9020 (2%), and Eusolex OCR (1%). The photolyase preparation contained 1% w/w of photolyase derived from the cyanobacterium *A*. *nidulans* in a liposomal preparation. The endonuclease preparation contained M. luteus-derived endonuclease incapsulated in liposomes (1% w/w). The vehicle (placebo) was a commercially available moisturizer base.

### Solar simulator

Solar-simulated radiation was produced by an Oriel solar simulator (Model 81292, L.O.T. Oriel, Leatherhead, UK) containing a 1 kW xenon arc lamp with two dichroic mirrors, a collimator, and a 1-mm WG320 filter. The optical design of this particular solar simulator gives a field of even irradiance (290-400 nm) at the skin surface when positioned 11 cm from the source, of which about 10% is UVB (280-320 nm) and the remainder UVA. The spectral irradiance was measured with an OL754 spectroradiometer (Optronics, Orlando, FL, USA), calibrated for wavelength and intensity against standard lamps. The spectroradiometer was used to calibrate a handheld IL700 radiometer (International Light, Newburyport, MA, USA), which was then used to rapidly monitor lamp output on a daily basis.

### Irradiation and treatment protocol

Two weeks before the test irradiations, the minimal erythema dose (MED) was determined for each individual for solar-simulated UVR (290-400 nm) and expressed in mJ/cm2 by using a light-proof adhesive-backed foil template that were sequentially uncovered to deliver quantities of UVR above and below the expected MED of skin phototype II individuals for solar-simulating UVR. The sites were examined 24 h after irradiation and the MED was determined as the site that showed minimal, uniform perceptible erythema. Before irradiation, six circular areas (10 mm diameter) were marked out on the non-exposed lower back of each participant. One site was left untreated (site 1: negative control), whereas the remaining 5 sites (designated sites 2-6) were exposed to solar-simulated UVR at 3 times the MED on four consecutive days. Site 2 received ssUVR only (site 2: positive control), whereas the following products were applied to sites 3-6, respectively: vehicle (moisturizer base cream; applied both thirty minutes before and immediately after each irradiation; site 3); a traditional sunscreen (SS, SPF AT LEAST 50) thirty minutes before irradiation and a vehicle immediately after irradiation (site 4); a SS thirty minutes before irradiation and an endonuclease preparation immediately after irradiation (site 5); a SS plus photolyase thirty minutes before irradiation arid an endonuclease preparation immediately after irradiation (site 6). Twenty-four hours after the last exposure to UVR, skin specimens were obtained through a 4-mm punch biopsy from all sites for molecular analyses.

### DNA extraction and measurements of telomere length in skin biopsies

The skin biopsy specimens were cleaved in half, and one piece was thawed at room temperature, minced, and lysed by three cycles of freezing (in an ethanol-dry-ice bath) and thawing (at 95°C). Samples were digested for 12 h at 60°C with proteinase K in 100 mmol/liter Tris-HCl (pH 7.4), 150 mmol/L NaCl, and 10 mmol/L EDTA (pH 8.0). Proteinase K was heat inactivated at 95°C for 10 min, and homogenates were extracted using the Puregene DNA Isolation kit (Gentra Systems, Minneapolis, MN, USA). The kit contains two main reagents: cell lysis and protein precipitation solutions. In brief, DNA was extracted from homogenates using a lysis buffer solution and then treated with RNase A. The kit removes proteins using a precipitation solution, followed by 2-propanol to pellet the DNA. Telomere length was measured as abundance of telomeric template (T) vs a single gene copy (S) by quantitative real-time PCR as previously described with slight modifications. For the T/S analysis, a 5 µL-aliquot with 20 ng of DNA and 10 µL of master mix were added to each sample well. For each standard curve one reference DNA sample was serially diluted in H₂O by 1.68-fold per dilution to produce five concentrations of DNA ranging from 30 ng to 2 ng in 5 µL. The composition of T and S PCRs were identical except for the oligonucleotide primers. Telomere and single copy gene (36B4) were analyzed in the same plate in order to reduce inter-assay variability. Measurements were performed in triplicate and reported as T/S ratio in respect to a calibrator sample. The same calibrator sample was used in all runs to allow comparison across runs. Every PCR was carried out on a BioRad iQ5 Cycler (BioRad, Hercules, CA, USA). The PCR protocol for the amplicons begun with a 95°C incubation for 10 min followed by 30 cycles of 95°C for 5 sec, 57°C for 15 sec and 72 °C for 20 sec. The melting curve values for telomere length and single copy gene corresponded to the expected values. The coefficients of variation within duplicates of the telomere and single-gene assay were 2.1% and 1.8% respectively.

### RNA extraction and assessment of c-FOS expression in skin biopsies

RNA from skin samples was isolated using the RNeasy Mini Kit (Qiagen, Valencia, CA, USA) according to the manufacturer's instructions. Integrity of RNA was assessed with agarose gel electrophoresis, and RNA quantity was measured by spectrophotometry. A 1 µg amount of RNA was reverse transcribed using the iScript cDNA Synthesis Kit (BioRad) according to the manufacturer's instructions. cDNA was stored at -20°C. In brief, a 25 µL reaction solution consisted of iQ SYBR Green Supermix (BioRad), forward and reverse primers (final concentration 400 nM each), and cDNA mixture (40 ng). The primers for c-FOS were: forward 5'-TCTCTTACTACCACTCACCC-3' and reverse 5'TGGAGTGTATCAGTCAGCTC-3' as described previously. To control for variations in RNA quality and quantity, the expression of the gene of interest was normalized to the expression of hypoxanthine phosphoribosyltransferase-1 (HPRT1) as a housekeeping gene. mRNA expression levels were calculated according to the following formula: 2-ΔCT, where ΔCT (sample) was defined as CT (c-FOS) - CT (HPRT1).

### Data analysis

Statistical analyses were carried out using SPSS for Windows, version 14.0 (SPSS Inc., Chicago, IL, USA). Because the Shapiro-Wilk test provided evidence that the data were normally distributed, only parametric statistics were used. Continuous variables are presented as means and standard deviations, whereas categorical variables are given as counts. One-way ANOVA followed by Newman-Keuls multiple-comparison post-hoc test was used to analyze intergroup differences. Given the exploratory nature of the study, no Bonferroni correction was used. A probability value <0.05 (two-tailed) was considered statistically significant.

### Telomers

As shown in figure 3, repeated irradiations (4 consecutive days) resulted in an equal reduction of telomeres length (T/S ratio) both at site 2 (UV radiations only) and at site 3 (UV + vehicle only) with 31% reduction compared to the non-irradiated site 1 (P<0.001).

Application of a SPF AT LEAST 50 sunscreen prior to exposure (site 4) resulted in a lesser reduction (10%) of telomeres length (P< 0.05 compared to site 2 and to site 3).

Remarkably, application of a solar screen with SPF AT LEAST 50 before the exposure and of composition ii. (comprising endonuclease) after exposure results in relevant further decrease of the telomere length reduction with respect to the application of sunscreen alone (+8.3% compared to site 4, P<0.05).

Application of composition i. (photolyase + SPF50 sunscreen) prior to exposure together with application of composition ii after exposure results in a telomere length substantially unchanged with respect to the non-irradiated site 1 and significantly higher (+23%) compared to site 5 (SPF50 sunscreen prior to exposure and composition ii. after exposure, P<0.001).

### FOS

### Telomers

As shown in figure 4, repeated irradiations (4 consecutive days) resulted in an equal increase in FOS gene expression (measured as ratio in arbitrary units with respect to a housekeeping gene) both at site 2 (UV radiations only) and at site 3 (UV + vehicle only) of 71% compared to base level (P<0.01 with respect to non-irradiated site 1).

Application of a SPF AT LEAST 50 sunscreen prior to exposition lead to a reduction of FOS expression of about 10% (P<0.05 compared to sites 2 and 3). However, application of a SPF AT LEAST 50 sunscreen prior to exposure and of composition ii. (endonuclease-comprising emulsion) after exposure (site 5) results in a relevant further reduction of FOS gene expression (-12% compared to site 4, P<0.05).

Application of composition i. (SPF AT LEAST 50 sunscreen and photolyase) prior to exposure followed by application of composition ii. after exposure (site 6) results in a FOS expression practically unchanged with respect to the non-irradiated site 1 and significantly lower (-19%) than site 5 (SPF AT LEAST 50 sunscreen prior to exposure and composition ii. after exposure, P<0.01).

### Application Project

<120> Title : COMPOSITIONS FOR PREVENTING AND REPAIRING SKIN AND OCULAR DAMAGES CAUSED BY UV RADIATIONS
<130> AppFileReference : P04327/EP
<140> CurrentAppNumber : 12756827.7
<141> CurrentFilingDate : 2012-07-05

### Sequence

<213> OrganismName : human
<400> PreSequenceString :
   tctcttacta ccactcaccc 20
<212> Type: DNA
<211> Length : 20
   SequenceName : SEQ ID N° 1_Forward primer for c-FOS
   SequenceDescription :

### Sequence

<213> OrganismName : human
<400> PreSequenceString :
   tggagtgtat cagtcagctc 20
<212> Type: DNA
<211> Length : 20
   SequenceName : SEQ ID N° 2_Reverse primner for c-FOS
   SequenceDescription :

## Claims

1. A kit comprising:
i. a sunscreen composition in form of a cream, of a solution or of an emulsion with sun protection factor at least 50 which comprises at least one UV-screening compound and liposomes including photolyase from *Anacystis nidulans;*
ii. a composition in form of a cream, of a solution or of an oil/water emulsion comprising liposomes including endonuclease from *Micrococcus luteus.*

2. The kit of claim 1 wherein the at least one UV-screening compound of composition i. is selected from the group consisting of methylene bis-benzotriazolyl tetramethylbuthylphenol, ethylhexyl methoxycinnamate, bis-ethylhexyloxyphenol methoxy phenyltriazine, butyl methoxydibenzoylmethane, 2-ethylhexyl-2-cyano-3,3-diphenylacrylate and mixtures thereof.

3. The kit of claim 1 or 2 wherein the composition i. comprises liposomes-included photolyase from *Anacystis nidulans* in a concentration between 0.1% and 5% w/w and the composition ii. comprises liposomes-included endonuclease from *Micrococcus luteus* in a concentration between 0.1% and 5% w/w.

4. The kit of claim 1 for use in the treatment and/or prevention of damages induced by exposure to UV radiations,
wherein
the treatment and/or prevention comprises the topical application, on the body areas to be exposed to the UV radiation, of composition i. prior to exposure to the radiations and the topical application of composition ii. on the same parts after exposure to UV radiation,
wherein
said damages are field cancerization induced by exposure to sunlight or to other sources of UV radiation, said field cancerization being in the form of actinic keratosis and squamous cell carcinoma in subjects with chronic, frequent and/or prolonged, exposure to sunrays and/or UV radiations.

5. The kit for use according to claim 4 in the prevention of damages caused by prolonged exposure to sunlight or to UV radiations without adequate protection (tanning addiction), by prolonged exposure to sunlight in outdoor workers, by photodynamic treatment (PDT), by exposure to sunlight of subjects with Fitzpatrick phototype II, suffering from immunodeficiency or photoallergy (photodermatitis), under prolonged cancer or steroidal therapy, or with tendency to multiple or recurrent basal-cell carcinoma.

## Patentansprüche

1. Ein Kit bestehend aus:
i. eine Sonnenschutzzusammensetzung in Form einer Creme, einer Lösung oder einer Emulsion mit einem Lichtschutzfaktor von mindestens 50, die mindestens eine UV-abschirmende Verbindung und Photolyase aus *Anacystis nidulans* enthaltende Liposomen umfasst;
ii. eine Zusammensetzung in Form einer Creme, einer Lösung oder einer Öl/ Wasser-Emulsion, die Endonuclease aus *Micrococcus luteus* enthaltende Liposomen umfasst.

2. Kit nach Anspruch 1, wobei die mindestens eine UV-abschirmende Verbindung der Zusammensetzung i. aus der Gruppe ausgewählt wird, die aus Methylenbisbenzotriazolyltetramethylbutylphenol, Ethylhexylmethoxycinnamat, Bisethylhexyloxyphenolmethoxyphenyltriazin, Butylmethoxydibenzoylmethan, 2-Ethylhexyl-2-cyano-3,3-diphenylacrylat und Mischungen davon besteht.

3. Kit nach Anspruch 1 oder 2, wobei die Zusammensetzung i. Photolyase aus *Anacystis nidulans* enthaltende Liposomen in einer Konzentration zwischen 0,1% und 5% w/w umfasst und die Zusammensetzung ii. Endonuclease aus *Micrococcus luteus* enthaltende Liposomen in einer Konzentration zwischen 0,1% und 5% w/w umfasst.

4. Kit nach Anspruch 1 zur Verwendung bei der Behandlung und/oder Vorbeugung von Schäden, die durch Bestrahlung mit UV-Strahlen hervorgerufen werden, wobei die Behandlung und/oder Vorbeugung die topische Anwendung der Zusammensetzung i. vor der Bestrahlung auf den der UV-Strahlung ausgesetzten Körperbereichen umfasst und die topische Anwendung der Zusammensetzung ii. an den gleichen Teilen nach der Einwirkung von UV-Strahlung umfasst, wobei die Schäden Feldkanzerisierung sind, die durch die Einwirkung von Sonnenlicht oder anderen UV-Strahlungsquellen induziert wird, wobei die Feldkanzerisierung in Form von aktinischer Keratose und Plattenepithelkarzinom bei Patienten mit chronischer, häufiger und/oder längerer Exposition gegenüber Sonnenstrahlen und/oder UV-Strahlung ist.

5. Kit zur Verwendung nach Anspruch 4 zur Vermeidung von Schäden, die durch längere Exposition gegenüber Sonnenlicht oder UV-Strahlung ohne ausreichenden Schutz (Bräunungssucht), durch längeres Sonnenlicht bei Outdoor-Arbeitern, durch photodynamische Behandlung (PDT), durch Sonnenlichteinwirkung bei Patienten verursacht werden, die den Fitzpatrick-Phototyp II haben, die unter Immunschwäche oder Photoallergie (Photodermatitis) leiden, die unter längerer Krebs- oder Steroidtherapie stehen oder die eine Neigung zu einem multiplen oder rezidivierenden Basalzellkarzinom haben.

## Revendications

1. Un kit comprenant :
i. une composition de protection solaire sous la forme d'une crème, d'une solution ou d'une émulsion avec un facteur de protection solaire d'au moins 50 qui comprend au moins un composé de criblage UV et des liposomes comprenant une photolyase provenant d'*Anacystis nidulans*;
ii. une composition sous la forme d'une crème, d'une solution ou d'une émulsion huile/eau comprenant des liposomes comprenant une endonucléase provenant de *Micrococcus luteus.*

2. Kit selon la revendication 1, dans lequel le au moins un composé de criblage UV de la composition i. est choisi dans le groupe constitué par le méthylène bis-benzotriazolyl tétraméthylbuthylphénol, l'éthylhexyl méthoxycinnamate, le bis-éthylhexyloxyphénol méthoxy phényltriazine, le butyl méthoxydibenzoylméthane, le 2-éthylhexyl-2-cyano-3,3-diphénylacrylate et leurs mélanges.

3. Kit selon la revendication 1 ou 2, dans lequel la composition i. comprend une photolyase contenant des liposomes provenant d'*Anacystis nidulans* à une concentration comprise entre 0,1% et 5% poids/poids et la composition ii. comprend une endonucléase contenant des liposomes provenant de *Micrococcus luteus* à une concentration comprise entre 0,1% et 5% poids/poids.

4. Kit selon la revendication 1 pour son utilisation dans le traitement et/ou la prévention des dommages induits par l'exposition aux rayonnements UV, dans lequel le traitement et/ou la prévention comprend l'application topique, sur les zones corporelles à exposer aux UV, de la composition i. avant l'exposition aux radiations et l'application topique de la composition ii. sur les mêmes parties après l'exposition au rayonnement UV, où lesdits dommages sont une cancérisation en champ induite par l'exposition à la lumière solaire ou à d'autres sources de rayonnement UV, ladite cancérisation en champ étant sous forme de kératose actinique et de carcinome épidermoïde chez des sujets avec une chronique, fréquente et/ou prolongée exposition aux rayons du soleil et/ou aux rayonnements UV.

5. Kit d'utilisation selon la revendication 4 pour la prévention des dommages causés par une exposition prolongée au soleil ou aux rayonnements UV sans protection adéquate (dépendance au bronzage), par une exposition prolongée au soleil chez les travailleurs extérieurs, par traitement photodynamique (PDT), par une exposition à la lumière du soleil de sujets atteints de phototype II de Fitzpatrick, de sujets souffrant d'immunodéficience ou de photoallergie (photodermatite), de sujets sous traitement prolongé par un cancer ou une thérapie stéroïdienne ou de sujets présentant une tendance à un carcinome basocellulaire multiple ou récurrent.
